# EUROPEAN PATENT SPECIFICATION

(11) **EP 1 991 179 B1**
(45) Date of publication and mention of the grant of the patent: **20.03.2013**
(21) Application number: 07751578.1
(22) Date of filing: 26.02.2007
(51) Int. Cl.: A61F 2/07, A61F 2/95

(54) **RETENTION OF STENTS**
STENTFIXIERUNG
RETENTION DE STENTS

(30) Priority: 27.02.2006 US 777106 P
(43) Date of publication of application: 19.11.2008
(73) Proprietor: William A. Cook Australia Pty. Ltd., Eight Mile Plains, Brisbane, Queensland 4113 (AU); Cook Medical Technologies LLC, Bloomington, IN 47404 (US)
(72) Inventor: HARTLEY, David, Ernest, Subiaco, Western Australia, 6008 (AU); RASMUSSEN, Erik, E., DK-4200 Slagelse (DK)
(74) Representative: Powell, Stephen David
(86) International application number: PCT/US2007/004826
(87) International publication number: WO 2007/100716

(56) References cited:
- EP-A- 1 029 517
- WO-A-03/101518
- WO-A-2005/058202
- US-A1- 2005 149 168

## Description

### Technical Field

This invention relates to stents and stent grafts, and more particularly to their retention on an introducer.

### Background of the Invention

For the endovascular introduction of stent grafts into the human or animal body, there have been proposed introducers or deployment devices which hold such stent grafts in a radially compressed or constrained condition on the introducer. Normally upon withdrawal of a sheath, an activation of a suitable release mechanism causes such a stent graft to be released into a body lumen.

Some stent grafts include an exposed proximally extended zigzag stent comprised of struts and bends between the struts. Retention of such a stent graft onto an introducer can be by retention of the bends of the zigzag exposed stent to the introducer via a release mechanism. It is important, however, that the bends and struts of the exposed stent are retained onto the introducer in a neat manner to enable the stent graft to be radially compressed or constrained into as small as possible region and to allow for release without tangling of the bends and struts of the exposed stent.

Aspects of the present invention seek to overcome or reduce one or more of the above problems. Aspects of the present invention also seek to provide the physician with a useful alternative construction.

Throughout this specification the term distal with respect to a portion of the aorta, a deployment device or a stent graft refers to the end of the aorta, deployment device or stent graft further away in the direction of blood flow away from the heart and the term proximal) refers to the portion of the aorta, deployment device or end of the stent graft nearer to the heart. When applied to other vessels similar terms such as caudal and cranial should be understood.

### Summary of the Invention

According to the present invention, there is provided a stent member comprising a tubular body portion and, extending from an end thereof, a plurality of struts with bends therebetween, a pair of struts and a bend between the pair of struts defining an apex a selected apex distance away from the tubular body portion, wherein adjacent apices are different apex distances from the tubular body portion, and that these adjacent apices are stactable are bereath the other.

EP-A-1029517 shows a stent member according to the preamble of claim 1.

The reference to a "pair" of struts does not necessarily imply that the struts of a pair are identical.

Preferably there are three apex distances being a shorter distance, a medium distance and a longer distance and that the three apex distances are consecutively placed around the exposed stent.

In a preferred embodiment the exposed stent can have twelve apices comprising four sets of the three apex distances consecutively spaced around the exposed stent.

There may be further included barbs on alternate struts of the exposed stent. The barbs are preferably distally extending.

To facilitate packing on an introducer and to prevent tangling of the barbs upon release, the barbs on alternate struts of the exposed stent may be spaced selected distances from their respective apices such that adjacent barbs are different distances from the tubular body portion.

According to an example not claimed there is provided a combination of a stent introducer and a stent member releasably retained thereon, the introducer comprising a trigger wire catheter and at least one trigger wire, the or each trigger wire extending in a loop from the trigger wire catheter, the stent member comprising a tubular body portion and, extending from one end of the tubular body portion, a plurality of struts with bends therebetween, a pair of struts and a bend between the pair of struts defining an apex a selected apex distance away from the tubular body portion, characterised in that adjacent apices are different apex distances from the tubular body portion, the stent member being retained on the introducer by the or each trigger wire passing through a plurality of apices at different apex distances from the tubular body portion.

Preferably the introducer has four trigger wires so that three bends of a twelve bend stent are retained by each trigger wire. There can be used a stent member with different numbers of proximal bends on the stent member and the introducer can have different numbers of trigger wires to retain the proximal end of the stent member to the introducer. For instance a stent with nine proximal bends can be retained on a delivery device using three trigger wires with three bends each retained by each wire. A stent with ten proximal bends can be retained on a delivery device using four trigger wires with two bends each retained by two of the wires and three bends each retained by the other two wires.

An advantage is thus provided by an arrangement for retaining the bends of a stent on an introducer by the use of trigger wires, in which adjacent bends stack up one next to the other, preferably three bends to a trigger wire, and thereby are retained in as small a volume as possible and can be released without problem.

Similarly, by placing the barbs spaced distances along alternate struts, the barbs have less tendency to engage with each other and cause problems. Furthermore, a more secure attachment to the surrounding body lumen is provided by such an arrangement.

### Brief Description of the Drawing

Preferred embodiments of the present invention will now be described, by way of example only, with reference to the accompanying drawings, of which:
Figure 1 shows a stent graft suitable for endovascular deployment and including an exposed zigzag stent at its proximal end according to one embodiment of the present invention;
Figure 2 shows a view of the proximal end of the stent of Figure 1 with the stent graft laid out flat;
Figure 3 shows the stent graft of Figures 1 and 2 in detail retained onto a stent graft introducer; and
Figures 4A, 4B and 4C show strut lengths and barb spacings for stent with different numbers of bends or points according to modifications of the embodiment of the invention.

### Detailed Description

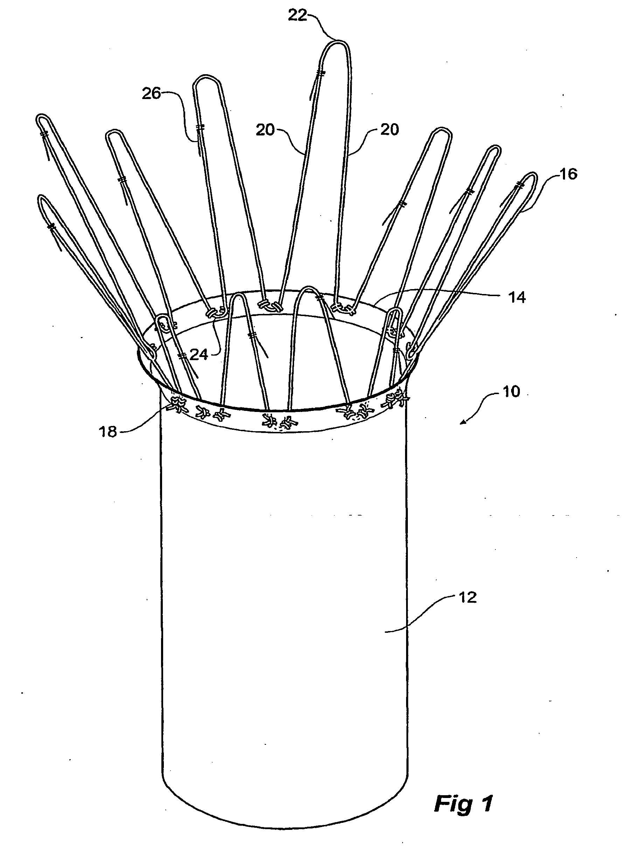

Referring to the drawings, and in particular Figure 1, it will be seen that a stent graft 10 has a tubular body 12 of a biocompatible graft material. At the proximal end 14 of the stent graft an exposed zigzag stent 16 is fastened to the tubular body 12 by stitching 18 and extends away from the tubular body. The exposed self expanding stent 16 is formed from struts 20 of a resilient wire such as stainless steel with proximal bends 22 and distal bends 24 between adjacent struts. The stitching 18 retaining the exposed stent to the proximal end of the stent graft is placed in the region of the distal bends 24. Barbs 26 are mounted onto alternate struts 20 with the point 27 of the barbs 26 extending distally. Further zig zag stents would normally be placed along the length of the stent graft but they have been omitted for clarity.

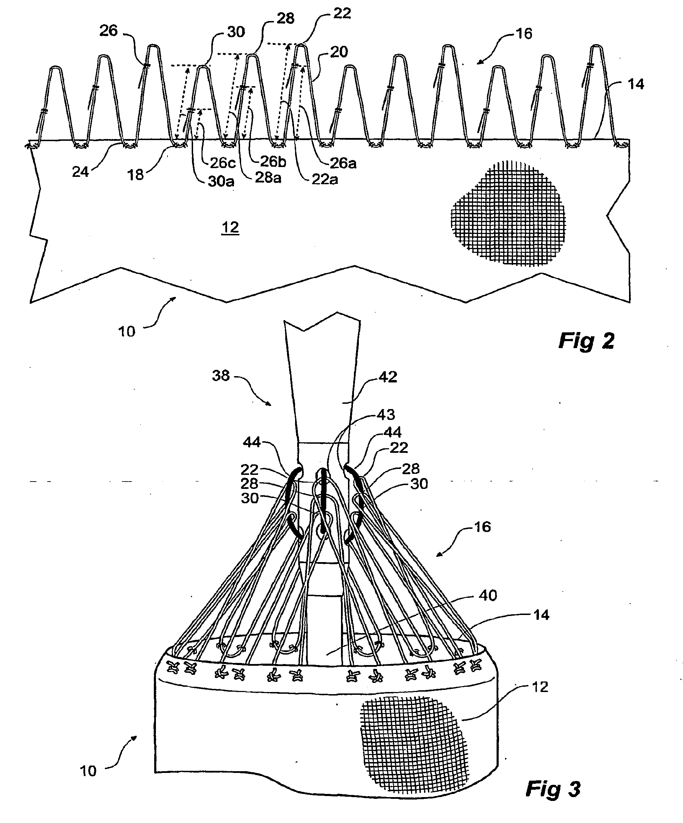

The proximal end 14 of the tubular body 12 of the stent graft 10 is shown in a laid out flat format (or developed view) in Figure 2.

It can be seen in Figure 2 that the bend 22 is the longest apex distance 22a from the tubular body 12, the adjacent bend 28 is a medium apex distance 28a from the tubular body 12 and the next adjacent bend 30 is the shortest apex distance 30a from the proximal end 14 of the tubular body 12. These three apex distances 22a, 28a and 30a are used consecutively around the proximal end of the tubular body.

It will also be noted that the barbs 26 on alternate struts 20 are spaced at different distances 26a, 26b and 26c from the proximal end 14 of the tubular body on adjacent alternate struts so as to assist in preventing adjacent barbs tangling or engaging with each other during introduction and release of the stent graft.

Figure 3 shows the stent graft of Figures 1 and 2 in detail retained onto a stent graft introducer before it is radially compressed within a sheath for delivery. Only part of the introducer is shown in Figure 3.

To retain the stent graft 10 onto an introducer 38 the exposed self expanding stent 16 is retained by trigger wires to the introducer. The introducer comprises a trigger wire catheter 40 extending to a nose cone dilator 42 with trigger wires 44 extending out of apertures 43 in the trigger wire catheter 40. In this embodiment there are four trigger wires 44. Each trigger wire 44 retains three proximal bends 22, 28 and 30 of the exposed stent 16.

It will be noted that adjacent individual bends 22, 28 and 30 of the exposed self expanding stent 16 are retained under a single trigger wire 44 with the bends being at different apex distances from the tubular body 12. Accordingly, they position neatly under the trigger wire and assist with stacking of the proximal end of the exposed stent 16 onto the introducer.

In a referred embodiment of the invention the exposed self expanding stent 16 has adjacent pairs of struts with lengths of 26, 25 and 24 millimetres from the proximal end 14 of the tubular body 12, and the barbs are placed onto the alternate struts at distances of 4 to 7mm, 7 to 10 mm and 10 to 13 mm from the respective bends of the exposed self expanding stent. Hence in one embodiment the barbs are spaced from the proximal end 14 of the tubular body 12 by distances of from 19 to 22 mm on the longest strut, 15 to 18 mm on the medium length strut and 11 to 14 mm on the shortest strut. All of these dimensions are measured along the struts and hence are at a slight angle to the longitudinal direction of the stent graft.

Various modifications may be made to the above-described embodiment. For example there may be only two apex distances. In other embodiments there may be four or more apex distances.

Barbs may be provided on every strut. In other embodiments, barbs are provided on fewer than half the struts. More than one barb may be provided on some or all of the struts.

A single trigger wire may extend through the loops of all the struts. Alternatively there may be two trigger wires, three trigger wires or more than four trigger wires. In general, a stent has c loops with d loops being arranged in each of b sets. Thus c = b x d and in general there will be b trigger wires, unless each trigger wire passes through the loops of more than one set.

The stent graft may comprise a plurality of stents arranged along its axis.

In a modification, a stent may be provided and deployed without graft material. The stent may be an end or exposed stent located at the end of any medical implant or prosthesis device.

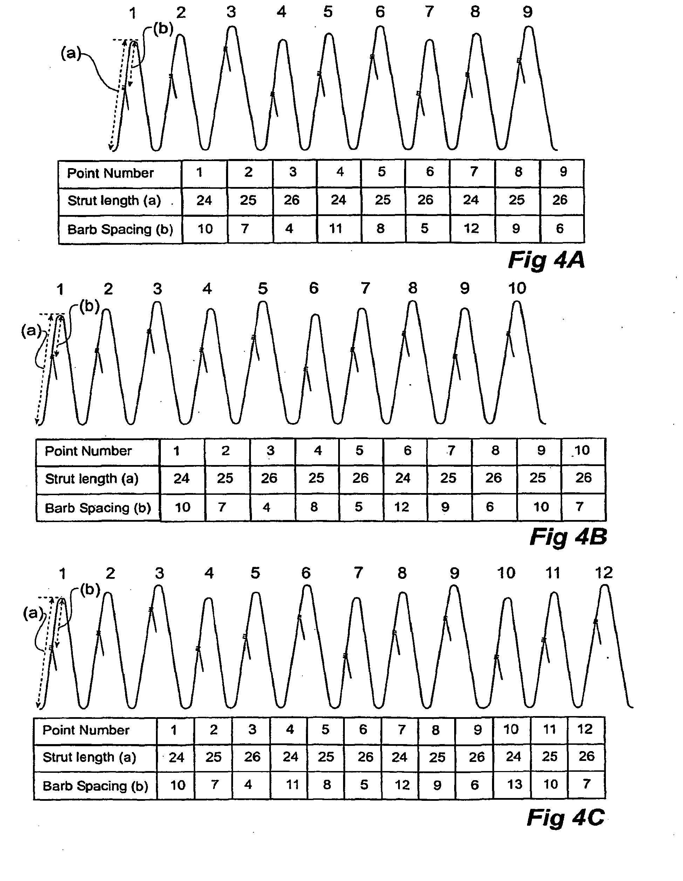

Figures 4A, 4B and 4C show strut lengths and barb spacings for stents with different numbers of bends or points according to further modifications of the invention. Figure 4A shows a stretched out stent with 9 proximal and 9 distal points or bends. Figure 4B shows a stretched out stent with 10 proximal and 10 distal points or bends. Figure 4C shows a stretched out stent with 12 proximal and 12 distal points or bends. The table accompanying each of Figures 4A, 4B and 4C show strut lengths and barb spacings for each point. All the dimensions are in millimetres. The length (a) in each of Figures 4A, 4B and 4C indicates the length of a strut of the stent and the length (b) in each of Figures 4A, 4B and 4C indicates the spacing of the barbs on alternate struts from the proximal point or bend. It will be noted that each strut is a different length than the strut on either side of it and that the barb spacing of adjacent barbs is different so that the barbs occupy as small a space as possible when the stent graft is compressed within a delivery sheath and are less likely to engage or tangle with each other.

## Claims

1. A stent member (10) comprising a tubular body portion and, extending from an end thereof, a plurality of struts (20) with bends (22, 24) therebetween, a pair of struts and a bend between the pair of struts defining an apex a selected apex distance away from the tubular body portion wherein adjacent apices are different apex distances from the tubular body portion **characterised in that** said adjacent apices stackable one beneath the other.

2. A stent member (10) according to Claim 1, wherein there are three apex distances being a shorter distance (30a), a medium distance (28a) and a longer distance (22a) each apex having a shorter apex distance being stackable beneath a respective apex having a medium apex distance which, in turn, is stackable beneath a respective apex having a longer apex distance.

3. A stent member (10) according to Claim 2, wherein the three apex distances (22a, 28a, 30a) are consecutively placed around the stent member (10).

4. A stent member (10) according to Claim 2, having twelve apices comprising four sets of the three apex distances (22a, 28a, 30a) consecutively placed around the stent member (10).

5. A stent member (10) according to any preceding Claim, further including barbs (26) on at least some of said struts.

6. A stent member according to claim 5, wherein barbs (26) are provided on alternate struts of the stent member (10).

7. A stent member (10) according to Claim 5 or 6, wherein the barbs (26) on different struts are spaced selected distances (26a, 26b, 26c) from the tubular body portion such that adjacent barbs (26) are different distances from the tubular body portion.

8. A stent member (10) according to any preceding Claim which is a self-expanding stent.

9. A stent member (10) according to any preceding Claim, which is a z stent.

10. A stent graft comprising a tubular body of graft-material (12) and one or more stents including a stent member (10) according to any preceding claim with at least its struts (20) exposed at one end (14) of the graft.

## Patentansprüche

1. Stentelement (10) mit einem rohrförmigen Körperabschnitt und mehreren sich von einem Ende davon erstreckenden Streben (20) mit dazwischenliegenden Biegungen (22, 24), wobei ein Paar Streben und eine Biegung zwischen dem Paar Streben einen Scheitel definieren, der mit einem gewählten Scheitelabstand vom rohrförmigen Körperabschnitt entfernt liegt, wobei benachbarte Scheitel verschiedene Scheitelabstände vom rohrförmigen Körperabschnitt haben, **dadurch gekennzeichnet, dass** die benachbarten Scheitel untereinander stapelbar sind.

2. Stentelement (10) nach Anspruch 1, wobei drei Scheitelabstände vorliegen, und zwar ein kürzerer Abstand (30a), ein mittlerer Abstand (28a) und ein längerer Abstand (22a), wobei jeder Scheitel mit einem kürzeren Scheitelabstand unter den jeweiligen Scheitel mit einem mittleren Scheitelabstand stapelbar ist, der wiederum unter einen jeweiligen Scheitel mit einem längeren Scheitelabstand stapelbar ist.

3. Stentelement (10) nach Anspruch 2, wobei die drei Scheitelabstände (22a, 28a, 30a) aufeinanderfolgend um das Stentelement (10) herum platziert sind.

4. Stentelement (10) nach Anspruch 2, mit zwölf Scheiteln, die vier Sätze mit drei Scheitelabständen (22a, 28a, 30a), die aufeinanderfolgend um das Stentelement (10) herum platziert sind, umfassen.

5. Stentelement (10) nach einem der vorhergehenden Ansprüche, ferner mit Haken (26) an mindestens einigen der Streben.

6. Stentelement (10) nach Anspruch 5, wobei Haken (26) an abwechselnden Streben des Stentelements (10) vorgesehen sind.

7. Stentelement (10) nach Anspruch 5 oder 6, wobei die Haken (26) an verschiedenen Streben unter Einhaltung von gewählten Abständen (26a, 26b, 26c) vom rohrförmigen Körperabschnitt beabstandet sind, so dass benachbarte Haken (26) unterschiedliche Abstände vom rohrförmigen Körperabschnitt haben.

8. Stentelement (10) nach einem der vorhergehenden Ansprüche, bei dem es sich um einen selbstexpandierenden Stent handelt.

9. Stentelement (10) nach einem der vorhergehenden Ansprüche, bei dem es sich um einen Z-Stent handelt.

10. Stentgraft mit einem rohrförmigen Körper aus Graft-Material (12) und einem oder mehreren Stents einschließlich eines Stentelements (10) nach einem der vorhergehenden Ansprüche, wobei mindestens dessen Streben (20) an einem Ende (14) des Grafts freiliegen.

## Revendications

1. Elément (10) de stent comportant une partie de corps tubulaire et, s'étendant depuis une extrémité de cette dernière, une pluralité d'entretoises (20) présentant des coudes (22, 24) entre ces dernières, une paire d'entretoises et un coude entre la paire d'entretoises délimitant un sommet à une distance de sommet sélectionnée du corps tubulaire, dans lequel des sommets adjacents sont à des distances de sommet différentes de la partie de corps tubulaire, **caractérisé en ce que** lesdits sommets adjacents sont empilables les uns en dessous des autres.

2. Elément (10) de stent selon la revendication 1, dans lequel il existe trois distances de sommets qui sont une distance (30a) plus courte, une distance (28a) moyenne et une distance (22a) plus longue, chaque sommet dont la distance de sommet est plus courte étant empilable en dessous d'un sommet respectif dont la distance de sommet est moyenne qui, à son tour, est empilable en dessous d'un sommet respectif dont la distance de sommet est plus longue.

3. Elément (10) de stent selon la revendication 2, dans lequel les trois distances (22a, 28a, 30a) de sommets sont situées consécutivement autour de l'élément (10) de stent.

4. Elément (10) de stent selon la revendication 2, présentant douze sommets comportant quatre jeux de trois distances (22a, 28a, 30a) de sommets situés autour de l'élément (10) de stent.

5. Elément (10) de stent selon l'une quelconque des revendications précédentes, comprenant en outre des crans (26) sur au moins certaines des entretoises.

6. Elément (10) de stent selon la revendication 5, dans lequel des crans (26) sont fournis sur des entretoises alternées de l'élément (10) de stent.

7. Elément (10) de stent selon la revendication 5 ou 6, dans lequel les crans (26) sur des entretoises différentes sont espacés à des distances (26a, 26b, 26c) sélectionnées de la partie de corps tubulaire de sorte que des crans (26) adjacents sont à des distances différentes de la partie de corps tubulaire.

8. Elément (10) de stent selon l'une quelconque des revendications précédentes qui est un stent auto-expansible.

9. Elément (10) de stent selon l'une quelconque des revendications précédentes, qui est un stent en z.

10. Greffe de stent comportant un corps tubulaire d'un matériau (12) de greffe et un ou plusieurs stents comprenant un élément (10) de stent selon l'une des revendications précédentes avec au moins ses entretoises (20) exposées à une extrémité (14) de la greffe.
